# EUROPEAN PATENT APPLICATION

(11) **EP 3 372 599 A1**
(43) Date of publication of application: **12.09.2018**
(21) Application number: 17159716.4
(22) Date of filing: 07.03.2017
(51) Int. Cl.: C07D 417/12, A61K 31/549, A61P 25/00

(54) **CRYSTALLINE FORM OF VERUBECESTAT**

(71) Applicant: Sandoz AG, 4056 Basel (CH)
(72) Inventor: LENGAUER, Hannes, 6250 Kundl (AT)
(74) Representative: Prüfer & Partner mbB Patentanwälte · Rechtsanwälte

(57) **Abstract**

The present invention relates to a crystalline form of verubecestat and to a process for its preparation. Furthermore, the invention relates to a pharmaceutical composition comprising said crystalline form of verubecestat, preferably in a predetermined and/or effective amount, and at least one pharmaceutically acceptable excipient. The pharmaceutical composition of the present invention can be used as a medicament, in particular for the treatment and/or prophylaxis Alzheimer's disease and symptoms thereof.

## Description

### FIELD OF THE INVENTION

The present invention relates to a crystalline form of verubecestat and to a process for its preparation. Furthermore, the invention relates to a pharmaceutical composition comprising said crystalline form of verubecestat, preferably in a predetermined and/or effective amount, and at least one pharmaceutically acceptable excipient. The pharmaceutical composition of the present invention can be used as a medicament, in particular for the treatment and/or prophylaxis of Alzheimer's disease and symptoms thereof.

### BACKGROUND OF THE INVENTION

Amyloid beta peptide is a primary component of beta amyloid fibrils and plaques, which are regarded as having a role in an increasing number of pathologies such as Alzheimer's disease. Amyloid beta peptides are short peptides which are made from the proteolytic break-down of the transmembrane protein called amyloid precursor protein (APP). In particular, they are made from the cleavage of APP by beta-secretase activity at a position near the N-terminus of amyloid beta peptide and by gamma-secretase activity at a position near the C-terminus of amyloid beta peptide. Beta site APP Cleaving Enzyme-1 (BACE-1) is regarded as the primary aspartyl protease responsible for the production of amyloid beta peptide by beta-secretase activity. The inhibition of BACE-1 has been shown to inhibit the production of amyloid beta peptide. Verubecestat has been shown to be a potent inhibitor of BACE-1 and BACE-2.

The compound verubecestat may be present in two tautomeric forms, which is on the one hand the "enamine" tautomer represented by the structure according to Formula Ia, and on the other hand the "imine" tautomer represented by the structure according to Formula Ib.

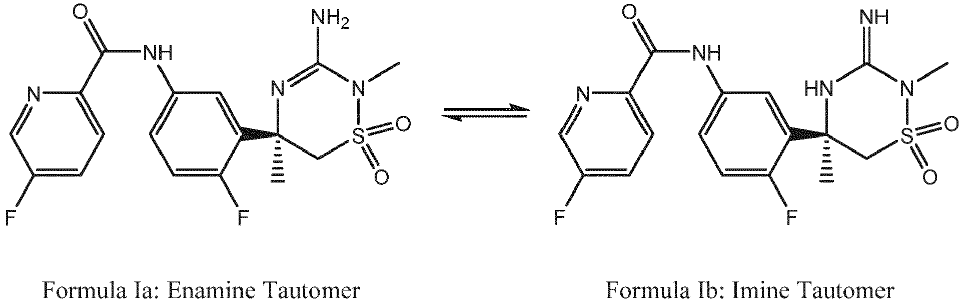

The "enamine" tautomer of verubecestat, which is shown above in Formula Ia may be chemically designated as *N*-[3-[(5*R*)-3-amino-5,6-dihydro-2,5-dimethyl-1,1-dioxido-2*H*-1,2,4-thiadiazin-5-yl]-4-fluorophenyl]-5-fluoro-2-pyridinecarboxamide. The "imine" tautomer which is shown above in Formula Ib may be chemically designated as 5-fluoro-*N*-[4-fluoro-3-[(5*R*)-tetrahydro-3-imino-2,5-dimethyl-1,1-dioxido-2*H*-1,2,4-thiadiazin-5-yl]phenyl]-2-pyridinecarboxamide.

WO 2011/044181 A1 discloses the compound verubecestat and its synthesis.

An anhydrous crystalline Form 1 of verubecestat free base and a crystalline Form 1 of verubecestat tosylate salt are disclosed in WO 2016/025364 A1. According to the application, Form 1 of verubecestat free base is obtained by adding n-heptane optionally followed by adding seed crystals to a solution of verubecestat in ethyl acetate at a temperature of 45-50 °C. Single crystals of Form 1 free base were obtained by solvent evaporation from ethyl acetate according to the application.

WO 2016/053767 A1 discloses a hemihydrate and a monohydrate of verubecestat free base as well as a crystalline Form 2 and a monohydrate of the tosylate salt. The hydrated forms disclosed therein are not suitable for the use in pharmaceutical processes, which involve the evolution of heat such as milling, drying or granulation processes etc. since they tend to dehydrate (e.g. lose their crystal water) already at moderate temperatures, as it can be seen from the DSC and TGA curves of the hemihydrate in Figures 8 and 9 of WO 2016/053767 A1. Moreover hydrates tend to dehydrate when exposed to low-humidity conditions.

Different solid state forms of an active pharmaceutical ingredient often possess different properties. Differences in the physicochemical properties of solid state forms can be important for the improvement of pharmaceutical compositions, for example, pharmaceutical formulations with improved dissolution profile or with improved stability or shelf-life can become accessible due to an improved solid state form of an active pharmaceutical ingredient. Also processing or handling of the active pharmaceutical ingredient during the formulation process may be improved. New solid state forms of an active pharmaceutical ingredient can thus have desirable processing properties. They can be easier to handle, better suited for storage, and/or allow for better purification, compared to previously known solid state forms. For example, salt formation is a usual means for improving the solubility and dissolution rate of low-soluble drug substances. Since verubecestat possesses high solubility and high permeability and therefore can be assigned to class 1 of the (Biopharmaceutical Classification System (BCS), salt formation is not required in order to improve the oral bioavailability of verubecestat.

However, it is desirable to use the thermodynamically most stable form of verubecestat free base, since the sudden appearance or disappearance of a metastable polymorph can present a problem in pharmaceutical development. Similarly, serious pharmaceutical consequences arise if transformation occurs in a dosage form, e.g. upon storage. It is therefore desirable to use a stable polymorph of an active pharmaceutical ingredient for the preparation of a drug product. Hence, there is a strong need for the provision of a solid form of verubecestat which is thermodynamically stable and does not undergo phase transformation during pharmaceutical processing or storage. There is also a strong need for the provision of a pharmaceutical composition comprising a solid form of verubecestat, which is stable at the conditions typically encountered during pharmaceutical processing and storage. The thermodynamically most stable solid form in particular can ensure reliable efficacy and safety for the whole duration of a pharmaceutical composition's shelf-life.

### SUMMARY OF THE INVENTION

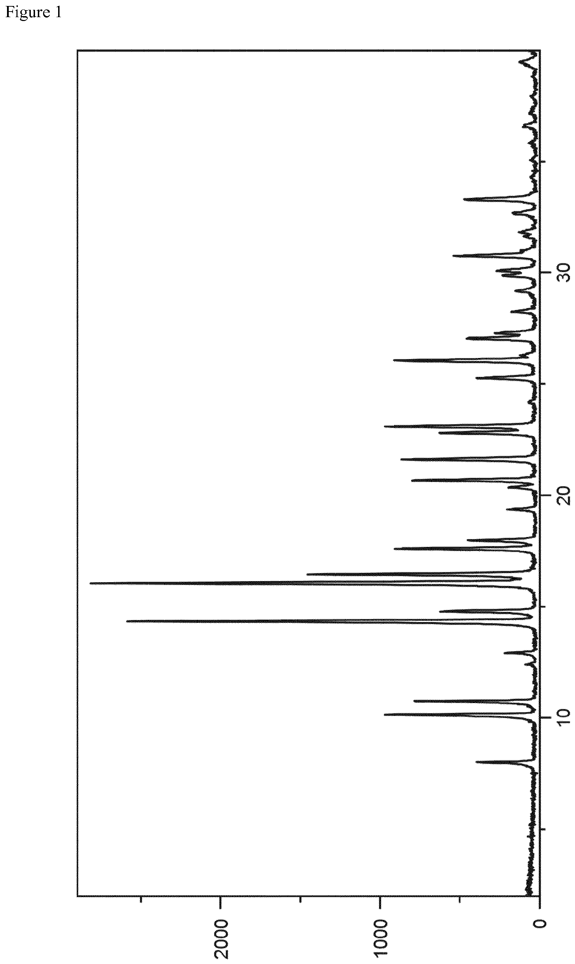

The inventors of the present invention surprisingly identified a polymorph of verubecestat free base, also designated as Form 2 hereinafter, which is thermodynamically more stable than the known Form 1 described in WO 2016/025364 A1 regardless of the temperature. Aspects, advantageous features and preferred embodiments of the present invention are summarized in the following items:
1) An anhydrous crystalline form of verubecestat (Form 2) characterized by having a powder X-ray diffractogram comprising reflections at 2-Theta angles of (14.8 ± 0.2)°, (16.1 ± 0.2)° and (26.0 ± 0.2)°, when measured at a temperature in the range of from 20 to 30 °C with Cu-Kalpha_{1,2} radiation having a wavelength of 0.15419 nm.
2) An anhydrous crystalline form of verubecestat (Form 2) characterized by having a powder X-ray diffractogram comprising reflections at 2-Theta angles of (14.3 ± 0.2)°, (14.8 ± 0.2)°, (16.1 ± 0.2)° and (26.0 ± 0.2)°, when measured at a temperature in the range of from 20 to 30 °C with Cu-Kalpha_{1,2} radiation having a wavelength of 0.15419 nm.
3) An anhydrous crystalline form of verubecestat (Form 2) characterized by having a powder X-ray diffractogram comprising reflections at 2-Theta angles of (14.3 ± 0.2)°, (14.8 ± 0.2)°, (16.1 ± 0.2)°, (16.5 ± 0.2)° and (26.0 ± 0.2)°, when measured at a temperature in the range of from 20 to 30 °C with Cu-Kalpha_{1,2} radiation having a wavelength of 0.15419 nm.
4) An anhydrous crystalline form of verubecestat (Form 2) characterized by having a powder X-ray diffractogram comprising reflections at 2-Theta angles of (8.0 ± 0.2)°, (14.3 ± 0.2)°, (14.8 ± 0.2)°, (16.1 ± 0.2)°, (16.5 ± 0.2)° and (26.0 ± 0.2)°, when measured at a temperature in the range of from 20 to 30 °C with Cu-Kalpha_{1,2} radiation having a wavelength of 0.15419 nm.
5) An anhydrous crystalline form of verubecestat (Form 2) characterized by having a powder X-ray diffractogram comprising reflections at 2-Theta angles (8.0 ± 0.2)°, (10.8 ± 0.2)°, (14.3 ± 0.2)°, (14.8 ± 0.2)°, (16.1 ± 0.2)°, (16.5 ± 0.2)° and (26.0 ± 0.2)°, when measured at a temperature in the range of from 20 to 30 °C with Cu-Kalpha_{1,2} radiation having a wavelength of 0.15419 nm.
6) An anhydrous crystalline form of verubecestat (Form 2) characterized by having a powder X-ray diffractogram comprising reflections at 2-Theta angles of (8.0 ± 0.2)°, (10.1 ± 0.2)°, (10.8 ± 0.2)°, (14.3 ± 0.2)°, (14.8 ± 0.2)°, (16.1 ± 0.2)°, (16.5 ± 0.2)° and (26.0 ± 0.2)°, when measured at a temperature in the range of from 20 to 30 °C with Cu-Kalpha_{1,2} radiation having a wavelength of 0.15419 nm.
7) An anhydrous crystalline form of verubecestat (Form 2) characterized by having a powder X-ray diffractogram comprising reflections at 2-Theta angles of (8.0 ± 0.2)°, (10.1 ± 0.2)°, (10.8 ± 0.2)°, (14.3 ± 0.2)°, (14.8 ± 0.2)°, (16.1 ± 0.2)°, (16.5 ± 0.2)°, (21.6 ± 0.2)° and (26.0 ± 0.2)°, when measured at a temperature in the range of from 20 to 30 °C with Cu-Kalpha_{1,2} radiation having a wavelength of 0.15419 nm.
8) An anhydrous crystalline form of verubecestat (Form 2) characterized by having a powder X-ray diffractogram comprising reflections at 2-Theta angles of (8.0 ± 0.2)°, (10.1 ± 0.2)°, (10.8 ± 0.2)°, (14.3 ± 0.2)°, (14.8 ± 0.2)°, (16.1 ± 0.2)°, (16.5 ± 0.2)°, (17.6 ± 0.2)°, (21.6 ± 0.2)° and (26.0 ± 0.2)°, when measured at a temperature in the range of from 20 to 30 °C with Cu-Kalpha_{1,2} radiation having a wavelength of 0.15419 nm.
9) An anhydrous crystalline form of verubecestat (Form 2) characterized by having a powder X-ray diffractogram comprising reflections at 2-Theta angles of (8.0 ± 0.2)°, (10.1 ± 0.2)°, (10.8 ± 0.2)°, (14.3 ± 0.2)°, (14.8 ± 0.2)°, (16.1 ± 0.2)°, (16.5 ± 0.2)°, (17.6 ± 0.2)°, (20.7 ± 0.2)°, (21.6 ± 0.2)° and (26.0 ± 0.2)°, when measured at a temperature in the range of from 20 to 30 °C with Cu-Kalpha_{1,2} radiation having a wavelength of 0.15419 nm.
10) An anhydrous crystalline form of verubecestat (Form 2) characterized by having a powder X-ray diffractogram comprising reflections at 2-Theta angles of (8.0 ± 0.2)°, (10.1 ± 0.2)°, (10.8 ± 0.2)°, (14.3 ± 0.2)°, (14.8 ± 0.2)°, (16.1 ± 0.2)°, (16.5 ± 0.2)°, (17.6 ± 0.2)°, (20.7 ± 0.2)°, (21.6 ± 0.2)°, (23.1 ± 0.2)° and (26.0 ± 0.2)°, when measured at a temperature in the range of from 20 to 30 °C with Cu-Kalpha_{1,2} radiation having a wavelength of 0.15419 nm.
11) An anhydrous crystalline form of verubecestat (Form 2) characterized by having a powder X-ray diffractogram comprising reflections at 2-Theta angles of (14.8 ± 0.1)°, (16.1 ± 0.1)° and (26.0 ± 0.1)°, when measured at a temperature in the range of from 20 to 30 °C with Cu-Kalpha_{1,2} radiation having a wavelength of 0.15419 nm.
12) An anhydrous crystalline form of verubecestat (Form 2) characterized by having a powder X-ray diffractogram comprising reflections at 2-Theta angles of (14.3 ± 0.1)°, (14.8 ± 0.1)°, (16.1 ± 0.1)° and (26.0 ± 0.1)°, when measured at a temperature in the range of from 20 to 30 °C with Cu-Kalpha_{1,2} radiation having a wavelength of 0.15419 nm.
13) An anhydrous crystalline form of verubecestat (Form 2) characterized by having a powder X-ray diffractogram comprising reflections at 2-Theta angles of (14.3 ± 0.1)°, (14.8 ± 0.1)°, (16.1 ± 0.1)°, (16.5 ± 0.1)° and (26.0 ± 0.1)°, when measured at a temperature in the range of from 20 to 30 °C with Cu-Kalpha_{1,2} radiation having a wavelength of 0.15419 nm.
14) An anhydrous crystalline form of verubecestat (Form 2) characterized by having a powder X-ray diffractogram comprising reflections at 2-Theta angles of (8.0 ± 0.1)°, (14.3 ± 0.1)°, (14.8 ± 0.1)°, (16.1 ± 0.1)°, (16.5 ± 0.1)° and (26.0 ± 0.1)°, when measured at a temperature in the range of from 20 to 30 °C with Cu-Kalpha_{1,2} radiation having a wavelength of 0.15419 nm.
15) An anhydrous crystalline form of verubecestat (Form 2) characterized by having a powder X-ray diffractogram comprising reflections at 2-Theta angles (8.0 ± 0.1)°, (10.8 ± 0.1)°, (14.3 ± 0.1)°, (14.8 ± 0.1)°, (16.1 ± 0.1)°, (16.5 ± 0.1)° and (26.0 ± 0.1)°, when measured at a temperature in the range of from 20 to 30 °C with Cu-Kalpha_{1,2} radiation having a wavelength of 0.15419 nm.
16) An anhydrous crystalline form of verubecestat (Form 2) characterized by having a powder X-ray diffractogram comprising reflections at 2-Theta angles of (8.0 ± 0.1)°, (10.1 ± 0.1)°, (10.8 ± 0.1)°, (14.3 ± 0.1)°, (14.8 ± 0.1)°, (16.1 ± 0.1)°, (16.5 ± 0.1)° and (26.0 ± 0.1)°, when measured at a temperature in the range of from 20 to 30 °C with Cu-Kalpha_{1,2} radiation having a wavelength of 0.15419 nm.
17) An anhydrous crystalline form of verubecestat (Form 2) characterized by having a powder X-ray diffractogram comprising reflections at 2-Theta angles of (8.0 ± 0.1)°, (10.1 ± 0.1)°, (10.8 ± 0.1)°, (14.3 ± 0.1)°, (14.8 ± 0.1)°, (16.1 ± 0.1)°, (16.5 ± 0.1)°, (21.6 ± 0.1)° and (26.0 ± 0.1)°, when measured at a temperature in the range of from 20 to 30 °C with Cu-Kalpha_{1,2} radiation having a wavelength of 0.15419 nm.
18) An anhydrous crystalline form of verubecestat (Form 2) characterized by having a powder X-ray diffractogram comprising reflections at 2-Theta angles of (8.0 ± 0.1)°, (10.1 ± 0.1)°, (10.8 ± 0.1)°, (14.3 ± 0.1)°, (14.8 ± 0.1)°, (16.1 ± 0.1)°, (16.5 ± 0.1)°, (17.6 ± 0.1)°, (21.6 ± 0.1)° and (26.0 ± 0.1)°, when measured at a temperature in the range of from 20 to 30 °C with Cu-Kalpha_{1,2} radiation having a wavelength of 0.15419 nm.
19) An anhydrous crystalline form of verubecestat (Form 2) characterized by having a powder X-ray diffractogram comprising reflections at 2-Theta angles of (8.0 ± 0.1)°, (10.1 ± 0.1)°, (10.8 ± 0.1)°, (14.3 ± 0.1)°, (14.8 ± 0.1)°, (16.1 ± 0.1)°, (16.5 ± 0.1)°, (17.6 ± 0.1)°, (20.7 ± 0.1)°, (21.6 ± 0.1)° and (26.0 ± 0.1)°, when measured at a temperature in the range of from 20 to 30 °C with Cu-Kalpha_{1,2} radiation having a wavelength of 0.15419 nm.
20) An anhydrous crystalline form of verubecestat (Form 2) characterized by having a powder X-ray diffractogram comprising reflections at 2-Theta angles of (8.0 ± 0.1)°, (10.1 ± 0.1)°, (10.8 ± 0.1)°, (14.3 ± 0.1)°, (14.8 ± 0.1)°, (16.1 ± 0.1)°, (16.5 ± 0.1)°, (17.6 ± 0.1)°, (20.7 ± 0.1)°, (21.6 ± 0.1)°, (23.1 ± 0.1)° and (26.0 ± 0.1)°, when measured at a temperature in the range of from 20 to 30 °C with Cu-Kalpha_{1,2} radiation having a wavelength of 0.15419 nm.
21) The crystalline form of verubecestat (Form 2) as defined in any one of items 1 to 20, characterized by having a powder X-ray diffractogram comprising no reflection at a 2-Theta angle of (15.3 ± 0.2)°.
22) The crystalline form of verubecestat (Form 2) as defined in any one of items 1 to 20, characterized by having a powder X-ray diffractogram comprising no reflection at a 2-Theta angle of (15.26 ± 0.2)°.
23) An anhydrous crystalline form of verubecestat (Form 2) characterized by having a powder X-ray diffractogram essentially the same as shown in Figure 1 of the present invention, when measured at a temperature in the range of from 20 to 30 °C with Cu-Kalpha_{1,2} radiation having a wavelength of 0.15419 nm.
24) The crystalline form of verubecestat (Form 2) as defined in any one of the preceding items characterized by having a TGA curve showing a mass loss of not more than 0.5 w-%, based on the weight of the crystalline form, when measured from 25 to 180 °C at a heating rate of 10 K/min.
25) The crystalline form of verubecestat (Form 2) as defined in any one of items 1 to 23 characterized by having a TGA curve showing a mass loss of not more than 0.4 w-%, based on the weight of the crystalline form, when measured from 25 to 180 °C at a heating rate of 10 K/min.
26) The crystalline form of verubecestat (Form 2) as defined in any one of items 1 to 23 characterized by having a TGA curve showing a mass loss of not more than 0.3 w-%, based on the weight of the crystalline form, when measured from 25 to 180 °C at a heating rate of 10 K/min.
27) The crystalline form of verubecestat (Form 2) as defined in any one of items 1 to 23 characterized by having a TGA curve showing a mass loss of not more than 0.2 w-%, based on the weight of the crystalline form, when measured from 25 to 180 °C at a heating rate of 10 K/min.
28) The crystalline form of verubecestat (Form 2) as defined in any one of the preceding items, which is non-solvated.
29) The crystalline form of verubecestat (Form 2) as defined in any one of the preceding items characterized by having a DSC curve showing an endotherm with an onset temperature of about 179 °C, when measured at a heating rate of 10 K/min.
30) The crystalline form of verubecestat (Form 2) as defined in any one of items 1 to 28 characterized by having a DSC curve showing an endotherm with an onset temperature of (179 ± 2) °C, when measured at a heating rate of 10 K/min.
31) The crystalline form of verubecestat (Form 2) as defined in any one of items 1 to 28 characterized by having a DSC curve showing an endotherm with an onset temperature of (179 ± 1) °C, when measured at a heating rate of 10 K/min.
32) The crystalline form of verubecestat (Form 2) as defined in any one of items 1 to 28 characterized by having a DSC curve showing an endotherm with an onset temperature of 179.5 °C, when measured at a heating rate of 10 K/min.
33) The crystalline form of verubecestat (Form 2) as defined in any one of the preceding items characterized by having a DSC curve showing an endotherm with a peak temperature of about 181 °C, when measured at a heating rate of 10 K/min.
34) The crystalline form of verubecestat (Form 2) as defined in any one of items 1 to 32 characterized by having a DSC curve showing an endotherm with a peak temperature of (181 ± 2) °C, when measured at a heating rate of 10 K/min.
35) The crystalline form of verubecestat (Form 2) as defined in any one of items 1 to 32 characterized by having a DSC curve showing an endotherm with a peak temperature of (181 ± 1) °C, when measured at a heating rate of 10 K/min.
36) The crystalline form of verubecestat (Form 2) as defined in any one of items 1 to 32 characterized by having a DSC curve showing an endotherm with a peak temperature of 180.8 °C, when measured at a heating rate of 10 K/min.
37) The crystalline form of verubecestat (Form 2) as defined in any one of the preceding items characterized by having a DSC curve showing a heat of fusion of about 85 J/g, when measured at a heating rate of 10 K/min.
38) The crystalline form of verubecestat (Form 2) as defined in any one of items 1 to 36 characterized by having a DSC curve showing a heat of fusion of (85 ± 2) J/g, when measured at a heating rate of 10 K/min.
39) The crystalline form of verubecestat (Form 2) as defined in any one of items 1 to 36 characterized by having a DSC curve showing a heat of fusion of (85 ± 1) J/g, when measured at a heating rate of 10 K/min.
40) The crystalline form of verubecestat (Form 2) as defined in any one of items 1 to 36 characterized by having a DSC curve showing a heat of fusion of 85.1 J/g, when measured at a heating rate of 10 K/min.
41) A composition comprising the crystalline form of verubecestat as defined in any one of the preceding items, which composition is essentially free of any other physical form of verubecestat.
42) A composition comprising the crystalline form of verubecestat as defined in any one of items 1 to 40, characterized by comprising at most 20 weight% of any other physical form of verubecestat, based on the weight of the composition.
43) A composition comprising the crystalline form of verubecestat as defined in any one of items 1 to 40, characterized by comprising at most 10 weight% of any other physical form of verubecestat, based on the weight of the composition.
44) A composition comprising the crystalline form of verubecestat as defined in any one of items 1 to 40, characterized by comprising at most 5 weight% of any other physical form of verubecestat, based on the weight of the composition.
45) A composition comprising the crystalline form of verubecestat as defined in any one of items 1 to 40, characterized by comprising at most 2 weight% of any other physical form of verubecestat, based on the weight of the composition.
46) A composition comprising the crystalline form of verubecestat as defined in any one of items 1 to 40, characterized by comprising at most 1 weight% of any other physical form of verubecestat, based on the weight of the composition.
47) The composition according to any one of items 41 to 46, wherein one of the other physical form of verubecestat is anhydrous Form 1 characterized by having a powder X-ray diffractogram comprising reflections at 2-Theta angles of (7.6 ± 0.2)°, (12.5 ± 0.2)°, (14.0 ± 0.2)° (15.3 ± 0.2)°, when measured at a temperature in the range of from 20 to 30 °C with Cu-Kalpha_{1,2} radiation having a wavelength of 0.15419 nm.
48) The composition according to any one of items 42 to 47, wherein one of the other physical form of verubecestat is anhydrous Form 1 characterized by having a powder X-ray diffractogram comprising reflections at 2-Theta angles of 7.61°, 12.48°, 13.95° and 15.26°, when measured at a temperature in the range of from 20 to 30 °C with Cu-Kalpha_{1,2} radiation having a wavelength of 0.15419 nm.
49) The composition according to any one of items 41 to 46, wherein one of the other physical form of verubecestat is amorphous.
50) A composition comprising at least 90 w-% of the crystalline form of verubecestat as defined in any one of items 1 to 40, based on the weight of the composition.
51) The composition of item 50 comprising less than 5 w-% of any other physical form of verubecestat, based on the weight of the composition.
52) The composition of item 50 comprising less than 2 w-% of any other physical form of verubecestat, based on the weight of the composition.
53) A composition comprising at least 95 w-% of the crystalline form of verubecestat as defined in any one of items 1 to 40, based on the weight of the composition.
54) The composition of item 53 comprising less than 4 w-% of any other physical form of verubecestat, based on the weight of the composition.
55) The composition of item 53 comprising less than 2 w-% of any other physical form of verubecestat, based on the weight of the composition.
56) The composition according to any one of items 50 to 55, wherein the any other physical form of verubecestat is anhydrous Form 1 characterized by having a powder X-ray diffractogram comprising reflections at 2-Theta angles of (7.6 ± 0.2)°, (12.5 ± 0.2)°, (14.0 ± 0.2)° (15.3 ± 0.2)°, when measured at a temperature in the range of from 20 to 30 °C with Cu-Kalpha_{1,2} radiation having a wavelength of 0.15419 nm.
57) The composition according to any one of items 50 to 55, wherein the any other physical form of verubecestat is anhydrous Form 1 characterized by having a powder X-ray diffractogram comprising reflections at 2-Theta angles of 7.61°, 12.48°, 13.95° and 15.26°, when measured at a temperature in the range of from 20 to 30 °C with Cu-Kalpha_{1,2} radiation having a wavelength of 0.15419 nm.
58) The composition according to any one of items 50 to 55, wherein the any other physical form of verubecestat is amorphous.
59) A process for the preparation of the crystalline form of verubecestat as defined in any one of items 1 to 40 or the composition as defined in any one of items 41 to 58 comprising:
   (i) providing a solution comprising verubecestat and an aqueous organic solvent, wherein the organic solvent is acetone or acetonitrile;
   (ii) optionally seeding the solution provided in step (i) with the crystalline form as defined in any one of items 1 to 40;
   (iii) separating at least a part of the crystals obtained in step (ii) from the mother liquor.
60) The process of item 59, wherein the water content of the organic solvent is in the range of from 1 to 60 vol-%.
61) The process of item 59, wherein the water content of the organic solvent is in the range of from 1 to 50 vol-%
62) The process of item 59, wherein the water content of the organic solvent is 50 vol-%.
63) The process according to any one of items 59 to 62, wherein the verubecestat concentration of the solution provided in step (i) is in the range of from 10 to 125 g/L.
64) The process according to any one of items 59 to 62, wherein the verubecestat concentration of the solution provided in step (i) is in the range of from 15 to 120 g/L.
65) The process according to any one of items 59 to 62, wherein the verubecestat concentration of the solution provided in step (i) is in the range of from 50 to 100 g/L.
66) The process according to any one of items 59 to 62, wherein the verubecestat concentration of the solution provided in step (i) is 100 g/L.
67) The process according to any one of items 59 to 66, wherein the solution is prepared at a temperature in the range of from 20 to 30 °C.
68) The process according to any one of items 59 to 66, wherein the solution is prepared at a temperature in the range of from 40 to 80 °C.
69) The process according to any one of items 59 to 66, wherein the solution is prepared at a temperature in the range of from 40 to 60 °C.
70) The process according to any one of items 59 to 69 comprising filtering the solution obtained in step (i).
71) The process according to any one of items 59 to 70, wherein the amount of seed crystals applied in step (ii) is in the range of from 1 to 20 w-%, based on the amount of verubecestat applied in step (i).
72) The process according to any one of items 59 to 70, wherein the amount of seed crystals applied in step (ii) is in the range of from 2 to 10 w-%, based on the amount of verubecestat applied in step (i).
73) The process according to any one of items 59 to 70, wherein the amount of seed crystals applied in step (ii) is in the range of from 3 to 5 w-%, based on the amount of verubecestat applied in step (i).
74) The process according to any one of items 59 to 73, wherein step (ii) is carried out.
75) The process according to item 74, wherein the crystals are separated from the mother liquor by filtration, centrifugation, decantation or solvent evaporation.
76) The process according to item 75, wherein the crystals are separated from the mother liquor by filtration or centrifugation.
77) The process according to item 76, wherein the crystals are separated from the mother liquor by filtration.
78) The process according to any one of items 59 to 77, further comprising step (iv) washing the isolated crystals obtained in step (iii).
79) The process according to item 78, wherein the crystals are washed with an aqueous organic solvent, wherein the organic solvent is acetone or acetonitrile.
80) The process of item 79, wherein the water content of the organic solvent is in the range of from 1 to 60 vol-%.
81) The process of item 79, wherein the water content of the organic solvent is in the range of from 1 to 50 vol-%
82) The process of item 79, wherein the water content of the organic solvent is 50 vol-%.
83) The process according to any one of items 59 to 82, further comprising step (v) drying the crystals obtained in any one of steps (ii) to (iv).
84) The process according to item 83, wherein drying is performed at a temperature of 150 °C or less.
85) The process according to item 83, wherein drying is performed at a temperature of 100 °C or less.
86) The process according to item 83, wherein drying is performed at a temperature of 60 °C or less.
87) The process according to item 83, wherein drying is performed at a temperature of 40 °C or less.
88) The process according to item 83, wherein drying is performed at a temperature in the range of from 20 to 30 °C.
89) The process according to any one of items 83 to 88, wherein drying is performed for a period in the range of from 1 to 72 hours.
90) The process according to any one of items 83 to 88, wherein drying is performed for a period in the range of from 2 to 48 hours.
91) The process according to any one of items 83 to 88, wherein drying is performed for a period in the range of from 4 to 24 hours.
92) The process according to any one of items 83 to 88, wherein drying is performed for a period in the range of from 6 to 18 hours.
93) Use of the crystalline form of verubecestat as defined in any one of items 1 to 40 for the preparation of a pharmaceutical composition.
94) Use of the composition as defined in any one of items 41 to 58 for the preparation of a pharmaceutical composition.
95) A pharmaceutical composition comprising the crystalline form of verubecestat as defined in any one of items 1 to 40 and at least one pharmaceutically acceptable excipient.
96) The pharmaceutical composition as defined in item 95 comprising a predetermined and/or effective amount of the crystalline form of verubecestat as defined in any one of items 1 to 40.
97) A pharmaceutical composition comprising the composition as defined in any one of items 41 to 58 and at least one pharmaceutically acceptable excipient.
98) The pharmaceutical composition of item 97, comprising a predetermined and/or effective amount of the composition as defined in any one of items 41 to 58.
99) The pharmaceutical composition according to any one of items 95 to 98 which is an oral solid dosage form.
100) The pharmaceutical composition according to item 99, wherein the oral solid dosage form is a tablet.
101) The pharmaceutical composition according to item 99, wherein the oral solid dosage form is a capsule.
102) The pharmaceutical composition according to any one of items 95 to 101 for use as a medicament.
103) The pharmaceutical composition according to any one of items 95 to 101 for use in the treatment and/or prophylaxis of of Alzheimer's disease and symptoms thereof.

### Abbreviations

- PXRD: powder X-ray diffractogram
- DSC: differential scanning calorimetry
- TGA: thermogravimetric analyses
- RT: room temperature
- w-%: weight percent
- vol-%: volume percent

### Definitions

In the context of the present invention the following definitions have the indicated meaning, unless explicitly stated otherwise:
As used herein, the term "verubecestat" collectively and individually refers to the "enamine" tautomer herein represented by the structure according to Formula Ia which may be chemically designated as *N*-[3-[(5*R*)-3-amino-5,6-dihydro-2,5-dimethyl-1,1-dioxido-2*H*-1,2,4-thiadiazin-5-yl]-4-fluorophenyl]-5-fluoro-2-pyridinecarboxamide and to the "imine" tautomer herein represented by the structure according to Formula Ib, which may be chemically designated as 5-fluoro-*N*-[4-fluoro-3-[(5*R*)-tetrahydro-3-imino-2,5-dimethyl-1,1-dioxido-2*H-*1,2,4-thiadiazin-5-yl]phenyl]-2-pyridinecarboxamide. Those skilled in the art will appreciate that the relative amount(s) of each tautomeric form of verubecestat that is (or is not) present in a given sample may vary as influenced by the physical conditions in which the sample is present.

As used herein the term "room temperature" refers to a temperature in the range of from 20 to 30 °C.

As used herein, the term "measured at a temperature in the range of from 20 to 30 °C" refers to a measurement under standard conditions. Typically, standard conditions mean a temperature in the range of from 20 to 30 °C, i.e. at room temperature. Standard conditions can mean a temperature of about 22 °C. Typically, standard conditions can additionally mean a measurement under 20-80% relative humidity, preferably 30-70% relative humidity, more preferably 40-60% relative humidity and most preferably 50% relative humidity.

The term "Form 1" or "anhydrous Form 1" as used herein interchangeably, when talking about a physical form of verbubecestat refers to the anhydrous crystalline form of verubecestat, which is disclosed in WO 2016/025364 A1. Form 1 of verubecestat can be characterized by having a powder X-ray diffractogram comprising reflections at 2-Theta angles of (7.6 ± 0.2)°, (12.5 ± 0.2)°, (14.0 ± 0.2)° and (15.3 ± 0.2)°, when measured at a temperature in the range of from 20 to 30 °C with Cu-Kalpha_{1,2} radiation having a wavelength of 0.15419 nm. Alternatively Form 1 can be characterized by having a powder X-ray diffractogram comprising reflections at 2-Theta angles of 7.61°, 12.48°, 13.95° and 15.26°, when measured at a temperature in the range of from 20 to 30 °C with Cu-Kalpha_{1,2} radiation having a wavelength of 0.15419 nm.

The term "reflection" with regard to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see *"*Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta, preferably in the range of ± 0.1 2-Theta. Thus, a reflection that usually appears at 16.1° 2-Theta for example can appear between 15.9° and 16.3° 2-Theta, preferably between 16.0 and 16.2° 2-Theta on most X-ray diffractometers under standard conditions. Furthermore, one skilled in the art will appreciate that relative reflection intensities will show inter-apparatus variability as well as variability due to degree of crystallinity, preferred orientation, sample preparation and other factors known to those skilled in the art and should be taken as qualitative measure only.

Crystalline Form 2 of verubecestat may be referred to herein as being characterized by graphical data "as shown in" a Figure. Such data include, for example, PXRD, TGA and DSC. The person skilled in the art understands that factors such as variations in instrument type, response and variations in sample directionality, sample concentration and sample purity may lead to small variations for such data when presented in graphical form, for example variations relating to the exact peak positions and intensities. However, a comparison of the graphical data in the Figures herein with the graphical data generated for another or an unknown physical form and the confirmation that two sets of graphical data relate to the same crystal form is well within the knowledge of a person skilled in the art.

As used herein, the term "substantially free of any other physical form" with reference to a composition comprising a particular physical form of verubecestat means that the composition includes at most 20%, preferably at most 10%, more preferably at most 5%, even more preferably at most 2% and most preferably at most 1% by weight of any other physical form of verubecestat, based on the weight of the composition.

The term "physical form" as used herein refers to any crystalline and/or amorphous phase of a compound.

As used herein, the term "amorphous" refers to a solid form of a compound that is not crystalline. An amorphous compound possesses no long-range order and does not display a definitive X-ray diffraction pattern with reflections.

The term "hydrate" as used herein, refer to a crystalline solid were either water is cooperated in or accommodated by the crystal structure e.g. is part of the crystal structure or entrapped into the crystal (water inclusions). Thereby, water can be present in a stoichiometric or non-stoichiometric amount. When water is present in stoichiometric amount, the hydrate may be referred to by adding greek numeral prefixes. For example, a hydrate may be referred to as a hemihydrate or as a monohydrate depending on the water/verubecestat stoichiometry. The water content can be measured, for example, by Karl-Fischer (KF) titration.

The terms "anhydrous form" or "anhydrate" as used herein refer to a crystalline solid were no water is cooperated in or accommodated by the crystal structure. Anhydrous forms may still contain residual water, which is not part of the crystal structure but may be adsorbed on the surface or absorbed in disordered regions of the crystal. Typically, an anhydrous form does not contain more than 1.0 w-%, preferably not more than 0.5 w-% of water, based on the weight of the crystalline form.

The term "non-solvated" as used herein, when talking about a crystalline solid indicates that no organic solvent is cooperated in or accommodated by the crystal structure. Non-solvated forms may still contain residual organic solvents, which are not part of the crystal structure but may be adsorbed on the surface or absorbed in disordered regions of the crystal. Typically, a non-solvated form does not contain more than 1.0 w-%, preferably not more than 0.5 w-%, and most preferably not more than 0.1 w-% of organic solvents, based on the weight of the crystalline form.

As used herein, the term "mother liquor" refers to the solution remaining after crystallization of a solid from said solution.

A "predetermined amount" as used herein with regard to verubecestat refers to the initial amount of verubecestat used for the preparation of a pharmaceutical composition having a desired dosage strength of verubecestat.

The term "effective amount" as used herein with regard to verubecestat encompasses an amount of verubecestat, which causes the desired therapeutic and/or prophylactic effect.

As used herein, the term "about" means within a statistically meaningful range of a value. Such a range can be within an order of magnitude, typically within 10%, more typically within 5%, even more typically within 1% and most typically within 0.1 % of the indicated value or range. Sometimes, such a range can lie within the experimental error, typical of standard methods used for the measurement and/or determination of a given value or range.

### BRIEF DESCRIPTION OF THE FIGURES

**Figure 1****:** illustrates a representative PXRD of verubecestat Form 2 of the present invention. The x-axis shows the scattering angle in °2-Theta, the y-axis shows the intensity of the scattered X-ray beam in counts of detected photons.
**Figure 2****:** illustrates a comparison of a representative PXRD of crystalline Form 2 of verubecestat of the present invention (bottom) and a representative PXRD of Form 1 of verubecestat (top). The x-axis shows the scattering angle in °2-Theta. The powder X-ray diffractogram of Form 1 was shifted along the y-axis to separate the diffractograms for clarity. The y-axis is therefore arbitrary and was not labeled.
**Figure 3****:** illustrates a representative TGA curve of crystalline Form 2 of verubecestat of the present invention. The x-axis shows the temperature in degree Celsius (°C), the y-axis shows the mass (loss) of the sample in weight percent (w-%).

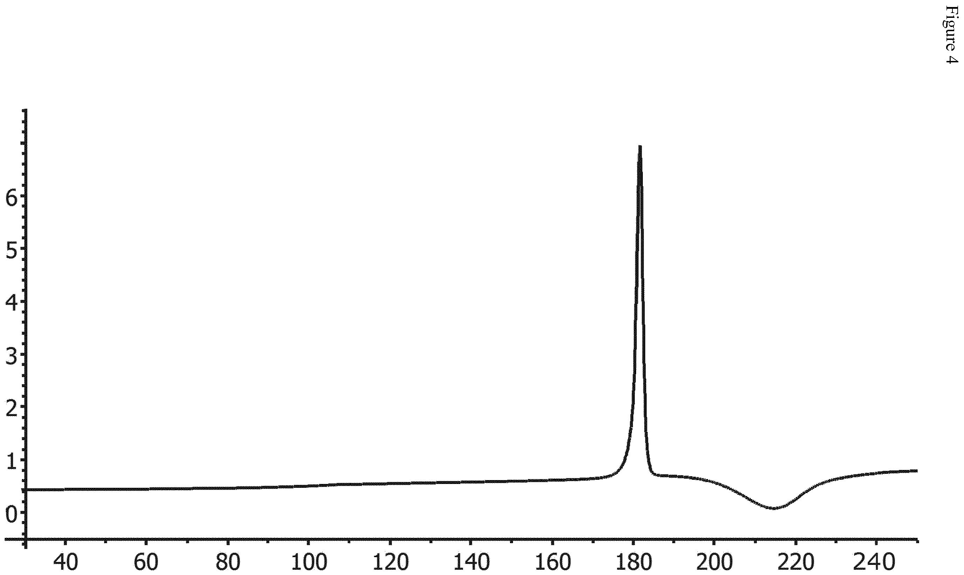

**Figure 4****:** illustrates a representative DSC curve of crystalline Form 2 of verubecestat of the present invention. The x-axis shows the temperature in degree Celsius (°C), the y-axis shows the heat flow rate in Watt per gram (W/g) with endothermic peaks going up.

### DETAILED DESCRIPTION OF THE INVENTION

The present invention provides an anhydrous crystalline form of verubecestat, herein also referred to as "Form 2".

The present inventors have surprisingly found that anhydrous Form 2 of the present invention is thermodynamically more stable than anhydrous Form 1 of WO 2016/025364 A1. The provision of a thermodynamically stable modification of verubecestat is highly desirable due to the fact that there is no risk of phase transitions such as polymorphic conversions and/or amorphization, which may occur during manufacture and/or storage of a drug product containing a metastable polymorph. Such transitions can in general have serious consequences for the pharmaceutical product with regard to safety and efficacy. Hence, Form 2 of the present invention is the favored solid form of verubecestat, to be used for the preparation of a pharmaceutical drug product. This is because the usage of Form 2 ensures a reliable safety and efficacy profile of a drug product containing Form 2 during the whole shelf-life of the product.

Crystalline Form 2 of verubecestat of the present invention may be characterized by analytical methods well known in the field of the pharmaceutical industry for characterizing solids. Such methods comprise but are not limited to PXRD, TGA and DSC. It may be characterized by one of the aforementioned analytical methods or by combining two or more of them. In particular, verubecestat Form 2 of the present invention may be characterized by any one of the following embodiments or by combining two or more of the following embodiments.

Hence, in a first aspect the invention relates to an anhydrous crystalline form of verubecestat (Form 2) characterized by having a powder X-ray diffractogram comprising reflections at 2-Theta angles of:
(14.8 ± 0.2)°, (16.1 ± 0.2)° and (26.0 ± 0.2)°; or
(14.3 ± 0.2)°, (14.8 ± 0.2)°, (16.1 ± 0.2)° and (26.0 ± 0.2)°; or
(14.3 ± 0.2)°, (14.8 ± 0.2)°, (16.1 ± 0.2)°, (16.5 ± 0.2)° and (26.0 ± 0.2)°; or
(8.0 ± 0.2)°, (14.3 ± 0.2)°, (14.8 ± 0.2)°, (16.1 ± 0.2)°, (16.5 ± 0.2)° and (26.0 ± 0.2)°; or
(8.0 ± 0.2)°, (10.8 ± 0.2)°, (14.3 ± 0.2)°, (14.8 ± 0.2)°, (16.1 ± 0.2)°, (16.5 ± 0.2)° and (26.0 ± 0.2)°; or
(8.0 ± 0.2)°, (10.1 ± 0.2)°, (10.8 ± 0.2)°, (14.3 ± 0.2)°, (14.8 ± 0.2)°, (16.1 ± 0.2)°, (16.5 ± 0.2)° and (26.0 ± 0.2)°; or
(8.0 ± 0.2)°, (10.1 ± 0.2)°, (10.8 ± 0.2)°, (14.3 ± 0.2)°, (14.8 ± 0.2)°, (16.1 ± 0.2)°, (16.5 ± 0.2)°, (21.6 ± 0.2)° and (26.0 ± 0.2)°; or
(8.0 ± 0.2)°, (10.1 ± 0.2)°, (10.8 ± 0.2)°, (14.3 ± 0.2)°, (14.8 ± 0.2)°, (16.1 ± 0.2)°, (16.5 ± 0.2)°, (17.6 ± 0.2)°, (21.6 ± 0.2)° and (26.0 ± 0.2)°; or
(8.0 ± 0.2)°, (10.1 ± 0.2)°, (10.8 ± 0.2)°, (14.3 ± 0.2)°, (14.8 ± 0.2)°, (16.1 ± 0.2)°, (16.5 ± 0.2)°, (17.6 ± 0.2)°, (20.7 ± 0.2)°, (21.6 ± 0.2)° and (26.0 ± 0.2)°; or
(8.0 ± 0.2)°, (10.1 ± 0.2)°, (10.8 ± 0.2)°, (14.3 ± 0.2)°, (14.8 ± 0.2)°, (16.1 ± 0.2)°, (16.5 ± 0.2)°, (17.6 ± 0.2)°, (20.7 ± 0.2)°, (21.6 ± 0.2)°, (23.1 ± 0.2)° and (26.0 ± 0.2)°;
when measured at a temperature in the range of from 20 to 30 °C with Cu-Kalpha_{1,2} radiation having a wavelength of 0.15419 nm.

In another embodiment, the present invention relates to an anhydrous crystalline form of verubecestat (Form 2) characterized by having a powder X-ray diffractogram comprising reflections at 2-Theta angles of:
(14.8 ± 0.1)°, (16.1 ± 0.1)° and (26.0 ± 0.1)°; or
(14.3 ± 0.1)°, (14.8 ± 0.1)°, (16.1 ± 0.1)° and (26.0 ± 0.1)°; or
(14.3 ± 0.1)°, (14.8 ± 0.1)°, (16.1 ± 0.1)°, (16.5 ± 0.1)° and (26.0 ± 0.1)°; or
(8.0 ± 0.1)°, (14.3 ± 0.1)°, (14.8 ± 0.1)°, (16.1 ± 0.1)°, (16.5 ± 0.1)° and (26.0 ± 0.1)°; or
(8.0 ± 0.1)°, (10.8 ± 0.1)°, (14.3 ± 0.1)°, (14.8 ± 0.1)°, (16.1 ± 0.1)°, (16.5 ± 0.1)° and (26.0 ± 0.1)°; or
(8.0 ± 0.1)°, (10.1 ± 0.1)°, (10.8 ± 0.1)°, (14.3 ± 0.1)°, (14.8 ± 0.1)°, (16.1 ± 0.1)°, (16.5 ± 0.1)° and (26.0 ± 0.1)°; or
(8.0 ± 0.1)°, (10.1 ± 0.1)°, (10.8 ± 0.1)°, (14.3 ± 0.1)°, (14.8 ± 0.1)°, (16.1 ± 0.1)°, (16.5 ± 0.1)°, (21.6 ± 0.1)° and (26.0 ± 0.1)°; or
(8.0 ± 0.1)°, (10.1 ± 0.1)°, (10.8 ± 0.1)°, (14.3 ± 0.1)°, (14.8 ± 0.1)°, (16.1 ± 0.1)°, (16.5 ± 0.1)°, (17.6 ± 0.1)°, (21.6 ± 0.1)° and (26.0 ± 0.1)°; or
(8.0 ± 0.1)°, (10.1 ± 0.1)°, (10.8 ± 0.1)°, (14.3 ± 0.1)°, (14.8 ± 0.1)°, (16.1 ± 0.1)°, (16.5 ± 0.1)°, (17.6 ± 0.1)°, (20.7 ± 0.1)°, (21.6 ± 0.1)° and (26.0 ± 0.1)°; or
(8.0 ± 0.1)°, (10.1 ± 0.1)°, (10.8 ± 0.1)°, (14.3 ± 0.1)°, (14.8 ± 0.1)°, (16.1 ± 0.1)°, (16.5 ± 0.1)°, (17.6 ± 0.1)°, (20.7 ± 0.1)°, (21.6 ± 0.1)°, (23.1 ± 0.1)° and (26.0 ± 0.1)°;
when measured at a temperature in the range of from 20 to 30 °C with Cu-Kalpha_{1,2} radiation having a wavelength of 0.15419 nm.

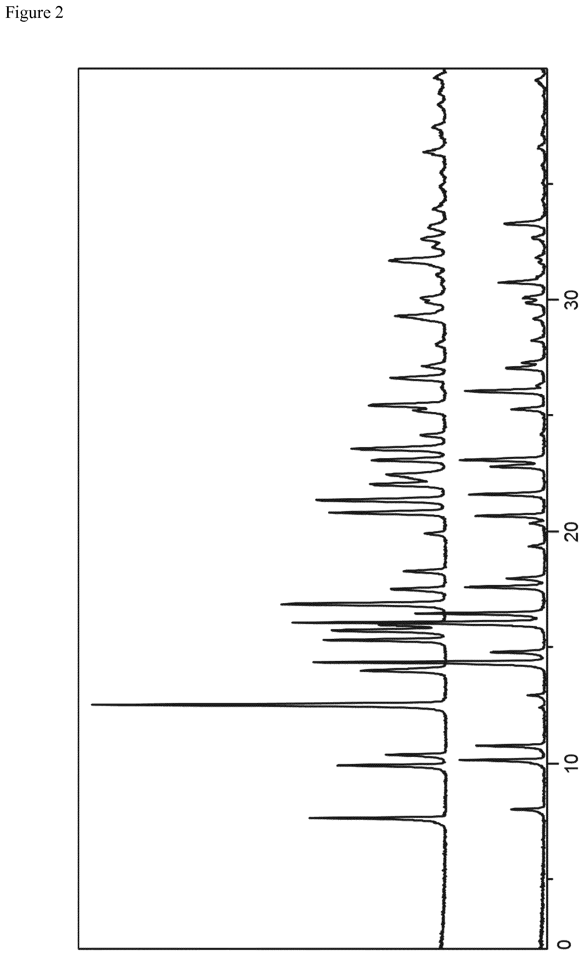

The PXRD of verubecestat Form 2 of the present invention can be clearly distinguished from the PXRD of Form 1 of WO 2016/025364 A1 (see also the PXRD overlay displayed in Figure 2 of the present invention). Form 2 for example shows characteristic reflections at (14.8 ± 0.2) and (26.0 ± 0.2)° 2-Theta, whereas Form 1 shows no reflection in the same ranges. According to page 29 of WO 2016/025364 A1 among the four most characteristic reflections of Form 1, one is the reflection at 15.26° 2-Theta. In contrast, Form 2 of the present invention shows no reflection in the range of (15.3 ± 0.2) 2-Theta.

Thus, in a another aspect, the present invention relates to an anhydrous crystalline form of verubecestat (Form 2) which can be characterized by having a powder X-ray diffractogram as described above, but not comprising a reflection at a 2-Theta angle of (15.3 ± 0.2)°, in particular not comprising a reflection at a 2-Theta angle of (15.26 ± 0.2)°.

In yet another embodiment, the present invention relates to an anhydrous crystalline form of verubecestat (Form 2) characterized by having a powder X-ray diffractogram essentially the same as shown in Figure 1 of the present invention, when measured at a temperature in the range of from 20 to 30 °C with Cu-Kalpha_{1,2} radiation having a wavelength of 0.15419 nm.

In a further embodiment, the present invention relates to an anhydrous crystalline form of verubecestat (Form 2) characterized by having a TGA curve showing a moss loss of not more than 0.5 w-%, preferably of not more than 0.4 w-%, more preferably of not more than 0.3 w-% and most preferably of not more than 0.2 w-%, based on the weight of the crystalline form, when heated from 25 to 180 °C at a heating rate of 10 K/min. In a preferred embodiment, the invention relates to an anhydrous crystalline form of verubecestat (Form 2) as defined herein, which is non-solvated.

In still another embodiment, the invention relates to an anhydrous crystalline form of verubecestat (Form 2) characterized by having a DSC curve showing an endotherm with an onset temperature of about 179 °C, preferably of (179 ± 2) °C, more preferably of (179 ± 1) °C, such as 179.5 °C, when measured at a heating rate of 10 K/min.

In another embodiment, the invention relates to an anhydrous crystalline form of verubecestat (Form 2) characterized by having a DSC curve showing an endotherm with a peak temperature of about 181 °C, preferably of (181 ± 2) °C, more preferably of (181 ± 1) °C, such as 180.8 °C, when measured at a heating rate of 10 K/min.

In a further embodiment, the invention relates to an anhydrous crystalline form of verubecestat (Form 2) characterized by having a DSC curve showing an endotherm with a heat of fusion of about 85 J/g, preferably of (85 ± 2) J/g, more preferably of (85 ± 1) J/g, such as 85.1 J/g, when measured at a heating rate of 10 K/min.

According to the DSC curve provided in Figure 2 of WO 2016/025364 A1 and the description on page 30 of WO 2016/025364 A1, anhydrous Form 1 of verubecestat shows a single sharp endotherm with an extrapolated onset temperature of 161.68 °C and a peak temperature of 164.47 °C. The heat of fusion was 80.22 J/g. The application teaches on page 30, lines 9 to 11 that the combination of melt temperature and heat of fusion can be used to distinguish crystalline anhydrous Form 1 of verubecestat from other crystal forms of verubecestat.

On top of that it is worthwhile mentioning that the melt temperature and heat of fusion can also be used to elucidate the thermodynamic relationship between different anhydrous polymorphic forms of a substance. For example, anhydrous crystalline Form 2 of verubecestat of the present invention shows a single sharp melting endotherm with an extrapolated onset temperature of 179.48 °C and a peak temperature of 180.75 °C. The heat of fusion was 85.14 J/g.

**Table 1: Comparison of DSC data**

| **DSC parameter** | **Anhydrous Form I of** WO 2016/025364 A1 | **Anhydrous Form II of present invention** |
|---|---|---|
| mp onset temperature | 161.86 °C | 179.48 °C |
| mp peak temperature | 164.47 °C | 180.75 °C |
| Heat of fusion | 80.22 J/g | 85.14 J/g |

As summarized in Table 1 above, anhydrous Form 2 of the present invention possesses the higher melting point and the higher heat of fusion compared to anhydrous Form 1. According to Burger and Ramberger's heat of fusion rule, Form 1 and Form 2 are monotropic with Form 2 being the thermodynamically stable form below its melting point.

The usage of the thermodynamically stable form is highly appreciated since polymorphic conversions, which may occur during the manufacturing process and/or during storage of a drug substance can be excluded, when the stable form is used. This ensures consistent quality and efficacy of a drug product.

In another aspect, the present invention relates to a composition comprising Form 2 of verubecestat of the present invention, which is essentially free of any other physical form of verubecestat. For example, a composition comprising Form 2 of verubecestat of the present invention comprises at most 20 weight%, preferably at most 10 weight%, more preferably at most 5 weight%, even more preferably at most 2 weight% and most preferably at most 1 weight% of any other physical form of verubecestat, based on the weight of the composition. Preferably, the any other physical form of verubecestat is Form 1 of WO 2016/025364 A1 or amorphous verubecestat.

In another embodiment, the invention relates to a composition comprising at least 90 w-% of the crystalline Form 2 of verubecestat as defined above, based on the total weight of the composition. Preferably, the composition comprises less than 5 w-%, such as less than 2 w-% of any other physical form of verubecestat. In a particular embodiment, the any other physical form of verubecestat is Form 1 or amorphous verubecestat.

In a further embodiment, the invention relates to a composition comprising at least 95 w-% of the crystalline Form 2 of verubecestat as defined above, based on the total weight of the composition. Preferably, the composition comprises less than 4 w-%, such as less than 2 w-% of any other physical form of verubecestat. In a particular embodiment, the any other physical form of verubecestat is Form 1 or amorphous verubecestat.

In another aspect, the present invention relates to a process for the preparation of the anhydrous crystalline Form 2 of verubecestat of the present invention or the composition comprising the anhydrous crystalline Form 2 of verubecestat as defined above comprising:
(i) providing a solution comprising verubecestat and an aqueous organic solvent, wherein the organic solvent is acetone or acetonitrile;
(ii) optionally seeding the solution provided in step (i) with anhydrous crystalline Form 2 of verubecestat of the present invention;
(iii)separating at least a part of the crystals obtained in step (ii) from the mother liquor;
(iv)optionally washing the isolated crystals obtained in step (iii);
(v) optionally drying the crystals obtained in any one of steps (ii) to (iv);

Suitable verubecestat starting materials, which may be applied in step (i) of the above described process can be prepared according to the procedures disclosed in one of the documents WO 2011/044181 A1, WO 2016/025359 A1, WO 2016/025364 A1 and WO 2016/053767 A1.

In a first step, the verubecestat starting material is dissolved in an aqueous organic solvent such as aqueous acetone or acetonitrile. In one embodiment, the water content of the organic solvent is in the range of from 1 to 60 vol%, preferably from 1 to 50 vol%, for example the water content of the organic solvent is 50 vol-%. The verubecestat concentration of the solution is preferably in the range of from about 10 to 125 g/L, more preferably from about 15 to 120 g/L and most preferably from about 50 to 100 g/L, for example the concentration is about 100 g/L. The solution is preferably prepared at room temperature but depending on the applied solvent and verubecestat concentration may also be prepared at elevated temperature for example at a temperature in the range of from about 40 to 80 °C, preferably from about 40 to 60 °C. The solution may optionally be filtered in order to remove any undissolved particles from the solution.

In order to initiate the crystallization of anhysdrous crystalline Form 2 of verubecestat the solution obtained in step (i) is preferably seeded with Form 2 crystals. The amount of seed crystals applied in step (ii) of the above described process is in the range of from 1 to 20 w-%, preferably from 2 to 10 w-% and most preferably from 3 to 5 w-%, based on the amount of verubecestat applied in step (i).

Form 2 seed crystals may be prepared by slow evaporation of an aqueous acetonitrile solution containing verubecestat, e.g. an aqueous acetonitrile solution wherein the water content of the organic solvent is in the range of from 1 to 60 vol%, preferably from 1 to 50 vol%, for example wherein the water content of the organic solvent is 50 vol-%. Preferably, the aqueous solvent is allowed to evaporate at room temperature, more preferably at atmospheric pressure. A concrete example for the Form 2 seed crystal preparation is provided in Example 2 herein.

After seeding the mixture is usually kept with or without stirring for a period in the range of from 1 to 48 hours, preferably from 2 to 24 hours until a sufficient amount of Form 2 crystals has been obtained.

Once verubecestat Form 2 is obtained in sufficient amount, at least a part of the crystals may be separated from the mother liquor. Preferably, the crystals are separated from the mother liquor by any conventional method such as filtration, centrifugation, solvent evaporation or decantation, more preferably by filtration or centrifugation and most preferably by filtration.

Optionally, in a further step the isolated crystals are washed with a suitable solvent, for example an aqueous organic solvent such as aqueous acetone or acetonitrile, wherein the water content of the organic solvent is in the range of from 1 to 60 vol%, preferably from 1 to 50 vol%, for example the water content of the organic solvent is 50 vol-%. Most preferably, the same aqueous solvent applied in step (i) of the above defined process is also used for washing.

The obtained crystals may then optionally be dried. Drying may be performed at a temperature of about 150 °C or less, preferably of about 100 °C or less, more preferably of about 60 °C or less and most preferably of about 40 °C or less. Typically, drying is performed at room temperature. Drying may be performed for a period in the range of from about 1 to 72 hours, preferably from 2 to 48 hours, more preferably from 4 to 24 hours and most preferably from 6 to 18 hours. Drying may be performed at ambient pressure and/or under reduced pressure. Preferably, drying is performed at a pressure of about 100 mbar or less, more preferably of about 50 mbar or less and most preferably of about 30 mbar or less, for example a vacuum of about 20 mbar or less.

In another aspect the present invention relates to the use of verubecestat Form 2 of the present invention as defined above for the preparation of a pharmaceutical composition.

In yet another aspect, the present invention relates to a pharmaceutical composition comprising crystalline Form 2 of verubecestat of the present invention, preferably in a predetermined and/or effective amount or the composition as defined above, and at least one pharmaceutically acceptable excipient. In a preferred embodiment, the pharmaceutical composition is an oral solid dosage form such as a tablet or a capsule.

In a further aspect, the present invention relates to the pharmaceutical composition as described above for use as a medicament.

In yet another aspect, the present invention relates to the pharmaceutical composition as described above for the treatment and/or prophylaxis of Alzheimer's disease and symptoms thereof.

### EXAMPLES

The following non-limiting examples are illustrative for the disclosure and are not to be construed as to be in any way limiting for the scope of the invention.

### Example 1: Preparation of anhydrous Form 2 of verubecestat

Amorphous verubecestat (500 mg, prepared according to Example 3 herein) was dissolved in aqueous acetone (5 mL, 50 vol-%) at room temperature. The obtained clear solution was seeded with verubecestat Form 2 (10 mg, prepared according to Example 2 herein) and allowed to stand at room temperature in a closed glass vial for 4 hours. The obtained solid was separated from the mother liquor by filtration followed by centrifugation using a centrifugal device (pore size 0.45 micrometer, Pall Life Sciences). The material obtained was analyzed by PXRD, TGA and DSC.

### Powder X-ray diffraction

PXRD was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. The diffractogram was recorded at a tube voltage of 45 kV and a tube current of 40 mA, applying a stepsize of 0.013° 2-theta with 40s per step (255 channels) in the angular range of 2° to 40° 2-Theta at ambient conditions. A typical precision of the 2-theta values is in the range of ± 0.2° 2-Theta, preferably of ± 0.1° 2-Theta. Thus, the diffraction peak of verubecestat Form 2 that appears for example at 16.1° 2-Theta can appear in the range of from 15.9 to 16.3° 2-Theta, preferably in the range of from 16.0 to 16.2° 2-Theta on most X-ray diffractometers under standard conditions.

A representative diffractogram of anhydrous Form 2 of verubecestat is displayed in Figure 1 herein. The corresponding reflection list is provided in Table 2 below.

**Table 2: PXRD reflections and corresponding relative intensities of anhydrous Form 2 of verubecestat in the range of from 2 to 30° 2-Theta; a typical precision of the 2-Theta values is in the range of ± 0.2° 2-Theta, preferably of ± 0.1° 2-Theta.**

| **Reflection position [° 2-Theta]** | **Relative intensity [%]** | **Reflection position [° 2-Theta]** | **Relative intensity [%]** |
|---|---|---|---|
| 8.0 | 13 | 20.7 | 28 |
| 10.1 | 34 | 21.6 | 29 |
| 10.8 | 27 | 22.8 | 22 |
| 12.4 | 2 | 23.1 | 33 |
| 12.9 | 6 | 24.2 | 1 |
| 14.3 | 93 | 25.3 | 13 |
| 14.8 | 21 | 26.0 | 32 |
| 16.1 | 100 | 26.3 | 3 |
| 16.5 | 53 | 27.0 | 15 |
| 17.6 | 31 | 27.3 | 9 |
| 18.0 | 15 | 28.2 | 5 |
| 19.4 | 6 | 29.2 | 4 |
| 20.3 | 6 | 29.8 | 7 |

### Thermogravimetric analysis

TGA was performed on a Mettler TGA/DSC 1 instrument. The sample (7.51 mg) was heated in a 100 microL aluminium pan closed with an aluminium lid, whereat the lid was automatically pierced at the beginning of the measurement. The sample was heated from 25 to 250 °C at a rate of 10 K/min. Nitrogen (purge rate 50 mL/min) was used as purge gas.

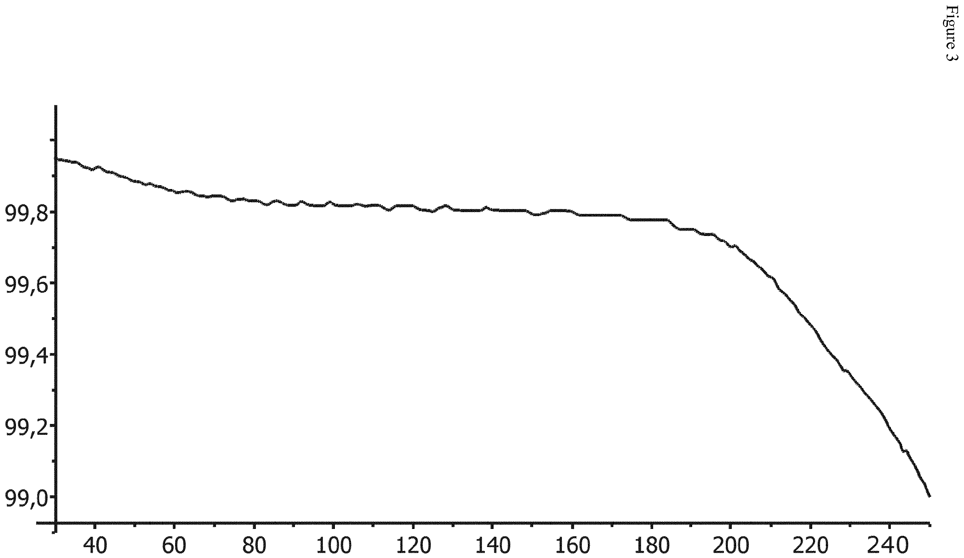

The TGA curve of Form 2, which is displayed in Figure 3 herein shows a mass loss of only about 0.2 w-% up to a temperature of about 180 °C, confirming the presence of a non-solvated and anhydrous form.

### Differential scanning calorimetry

Differential scanning calorimetry (DSC) was performed on a Mettler Polymer DSC R instrument. The sample (2.46 mg) was heated in a 40 microL aluminium pan with pierced aluminium lid from 25 to 250 °C at a rate of 10 K/min. Nitrogen (purge rate 50 mL/min) was used as purge gas.

Anhydrous crystalline Form 2 of verubecestat of the present invention shows a single sharp melting endotherm with an extrapolated onset temperature of 179.48 °C and a peak temperature of 180.75 °C. The heat of fusion was 85.14 J/g.

### Example 2: Preparation of verubecestat Form 2 seed crystals

Amorphous verubecestat (50 mg, prepared according to Example 3 herein) was dissolved in aqueous acetonitrile (3 mL, 50 vol-%) at room temperature. The obtained clear solution was filtered through a syringe filter (pore size 0.45 micrometer) and allowed to evapoarate on a watch glass at room temperature for 72 hours. The obtained solid was taken up in acetonitrile (3 mL) at room temperature and the solvent was again allowed to evaporate on the watch glass at room temperature for 24 hours to obtain anhydrous Form 2 of verubecestat.

### Reference example 3: Preparation of amorphous verubecestat

Verubecestat (5 g, e.g. prepared according to the process disclosed in WO 2016/025359 A1) was dissolved in dichloromethane (50 mL) at room temperature. Dichloromethane was removed on a rotary evaporator (400 mbar, 35 °C) and the obtained solid was dried at room temperature under vacuum (30 mbar) for 16 hours to obtain amorphous verubecestat.

## Claims

1. An anhydrous crystalline form of verubecestat (Form 2) **characterized by** having a powder X-ray diffractogram comprising reflections at 2-Theta angles of:
(14.8 ± 0.2)°, (16.1 ± 0.2)° and (26.0 ± 0.2)°; or
(14.3 ± 0.2)°, (14.8 ± 0.2)°, (16.1 ± 0.2)° and (26.0 ± 0.2)°; or
(14.3 ± 0.2)°, (14.8 ± 0.2)°, (16.1 ± 0.2)°, (16.5 ± 0.2)° and (26.0 ± 0.2)°;
when measured at a temperature in the range of from 20 to 30 °C with Cu-Kalpha_{1,2} radiation having a wavelength of 0.15419 nm.

2. The crystalline form of claim 1 **characterized by** having a powder X-ray diffractogram comprising no reflection at a 2-Theta angle of (15.3 ± 0.2)°, when measured at a temperature in the range of from 20 to 30 °C with Cu-Kalpha_{1,2} radiation having a wavelength of 0.15419 nm.

3. The crystalline form of claim 1 or 2 **characterized by** having a powder X-ray diffractogram essentially the same as shown in Figure 1 of the present invention, when measured at a temperature in the range of from 20 to 30 °C with Cu-Kalpha_{1,2} radiation having a wavelength of 0.15419 nm.

4. The crystalline form according to any one of the preceding claims **characterized by** having a thermogravimetric analysis curve showing a mass loss of not more than 0.5 w-%, based on the weight of the crystalline form, when measured from 25 to 180 °C at a heating rate of 10 K/min.

5. The crystalline form according to any one of the preceding claims, which is non-solvated.

6. The crystalline form according to any one of the preceding claims **characterized by** having a differential scanning calorimetry curve showing an endotherm with an onset temperature of (179 ± 2) °C, when measured at a heating rate of 10 K/min.

7. A composition comprising the crystalline form as defined in any one of the preceding claims and at most 20 weight%, 10 weight%, 5 weight%, 2 weight% or 1 weight% of any other physical form of verubecestat, based on the weight of the composition.

8. The composition according to claim 7, wherein one of the other physical forms of verubecestat is Form 1 **characterized by** having a powder X-ray diffractogram comprising reflections at 2-Theta angles of (7.6 ± 0.2)°, (12.5 ± 0.2)°, (14.0 ± 0.2)° and (15.3 ± 0.2)°, when measured at a temperature in the range of from 20 to 30 °C with Cu-Kalpha_{1,2} radiation having a wavelength of 0.15419 nm.

9. The composition according to claim 7 or 8, wherein one of the other physical forms is amorphous verubecestat.

10. A process for the preparation of the crystalline form as defined in any one of claims 1 to 6 or the composition as defined in any one of claims 7 to 9 comprising:
(i) providing a solution comprising verubecestat and an aqueous organic solvent, wherein the organic solvent is acetone or acetonitrile;
(ii) optionally seeding the solution provided in step (i) with the crystalline form as defined in any one of items 1 to 6;
(iii) separating at least a part of the crystals obtained in step (ii) from the mother liquor.

11. A pharmaceutical composition comprising the crystalline form as defined in any one of claims 1 to 6 or the composition as defined in any one of claims 7 to 9, and at least one pharmaceutically acceptable excipient.

12. The pharmaceutical composition of claim 13, wherein the pharmaceutical composition is an oral solid dosage form, optionally a tablet or a capsule.

13. The pharmaceutical composition of claim 13 or 14 for use in the treatment and/or prophylaxis of Alzheimer's disease and symptoms thereof.
